# EUROPEAN PATENT SPECIFICATION

(11) **EP 2 919 757 B1**
(45) Date of publication and mention of the grant of the patent: **05.10.2016**
(21) Application number: 13791976.7
(22) Date of filing: 12.11.2013
(51) Int. Cl.: A61K 9/08, A61K 9/10, A61K 36/00, A61K 41/00

(54) **A METHOD FOR PREPARING BIOACTIVE BOTANICAL COMPOSITIONS AND THE COMPOSITIONS MADE FROM SAID METHOD USING AN ELECTROMAGNETIC FIELD OF GREATER THAN 3 GHZ**
VERFAHREN ZUR ZUBEREITUNG VON BIOLOGISCH AKTIVEN BOTANISCHEN ZUSAMMENSETZUNGEN UND ZUSAMMENSETZUNGEN AUS DEM VERFAHREN UNTER VERWENDUNG EINES ELEKTROMNAGNETISCHEN FELDS VON MEHR ALS 3 GHZ
PROCÉDÉ DE PRÉPARATION DE COMPOSITIONS BOTANIQUES BIOACTIVES ET COMPOSITIONS FAITES À PARTIR DUDIT PROCÉDÉ EN UTILISANT UN CHAMP ÉLECTROMAGNÉTIQUE DE PLUS DE 3 GHZ

(30) Priority: 14.11.2012 US 201261726195 P; 22.02.2013 EP 13156315
(43) Date of publication of application: 23.09.2015
(73) Proprietor: ISP Investments Inc., Wilmington, DE 19805 (US)
(72) Inventor: KOGANOV, Michael, White Plains, New York 10601 (US)
(74) Representative: Kutzenberger Wolff & Partner
(86) International application number: PCT/EP2013/073565
(87) International publication number: WO 2014/076055

(56) References cited:
- US-A1- 2003 175 235
- US-A1- 2011 212 190
- J.Suhm, M.Möller, H.Linn: "New Development for Industrial Microwave Heating", International Scientific Colloquium Modelling for Electromagnetic Processing Hannover , 25 March 2003 (2003-03-25), pages 131-135, XP002695096, Retrieved from the Internet: URL:http://www.modlab.lv/publications/mep2 003/pdf/141.pdf [retrieved on 2013-04-10]

## Description

### Field of the Invention

The present invention relates to a process for the preparation of botanical fractions and to compositions made from said fractions.

### Background of the Invention

Over the past several decades, the pharmaceutical, cosmetic and personal care industries have embraced the use of plants and plant products in a variety of beneficial formulations and products. While this trend is expected to continue far into the future, there is a continuing need for higher quality botanical ingredients of enhanced purity and activity having fewer negative effects and which are solvent-free, and prepared by environmentally friendly and sustainable methods.

The industry as a whole has increased its support of efforts to develop and market "natural" formulations using a host of single and blended botanical ingredients that are currently available to the industry. In order to ensure quality, safety, and consistency, the cosmetic industry, as an example, has developed and implemented various standard operating procedures and strict specification controls for all incoming raw materials for use in cosmetic formulations. Many current botanical extracts fail to comply with the increasing controls and consistency parameters of the cosmetic industry. Current plant extraction methods limit product specification parameters leaving many windows of variability for quality, performance, and compatibility. In addition, current extraction methods fail to deliver the full spectrum of activities that exist within plant cells. Thus, the full potential of botanical-based cosmetic formulations is not being realized due to the inadequacy of the extraction methods for bioactive botanical cosmetic ingredients.

Many of the current methods for extracting bioactive components from plants involve techniques that are harmful to the plant tissue or the bioactive components of interest contained in that tissue, or both. Further, many of the current extraction and separation methods yield crude botanical extracts that contain biological or chemical contaminants that can cause a loss of bioactivity potency, increased cytotoxicity, and decreased shelf life. Further, in order to yield a more refined botanical extract, current extraction methods often require the use of harsh chemical solvents. Thus, there is a need for a method for preparing bioactive botanical compositions from plants that preserves the integrity of bioactive components and yields consistent results from lot-to-lot. Further, bioactive botanical compositions that are able to meet the industry standards with respect to shelf life, cytotoxicity, quality, and performance are needed in the cosmetic industry.

### Summary of the Invention

The present invention relates to a process for the preparation of botanical fractions from fresh plant biomass according to claim 1 and to compositions made from said fractions according to claim 14 or 15. The process comprises grinding (or maceration) and pressing fresh plant biomass in order to obtain an intracellular plant material (or plant cell juice) containing membrane fractions (containing nucleus, or chloroplasts, or chromoplasts, or mitochondria, or combinations of thereof), and treating said cell juice with an electromagnetic waves at a frequency effective to trigger separation of said membrane fraction from said cell juice in order to yield a cell cytoplasm/cytosole fraction (all residual components of cell juice) substantially-free from membrane fractions. The aforementioned treatment is performed such that the temperature of said cell juice during said treatment does not exceed 40°C.

The present invention also relates to botanical fractions derived from either the membrane fraction or the cytoplasm/cytosole fraction of fresh plants. The present invention further relates to processes for producing the botanical cosmetic compositions, as well as methods for using the compositions.

### Brief Description of the Drawings

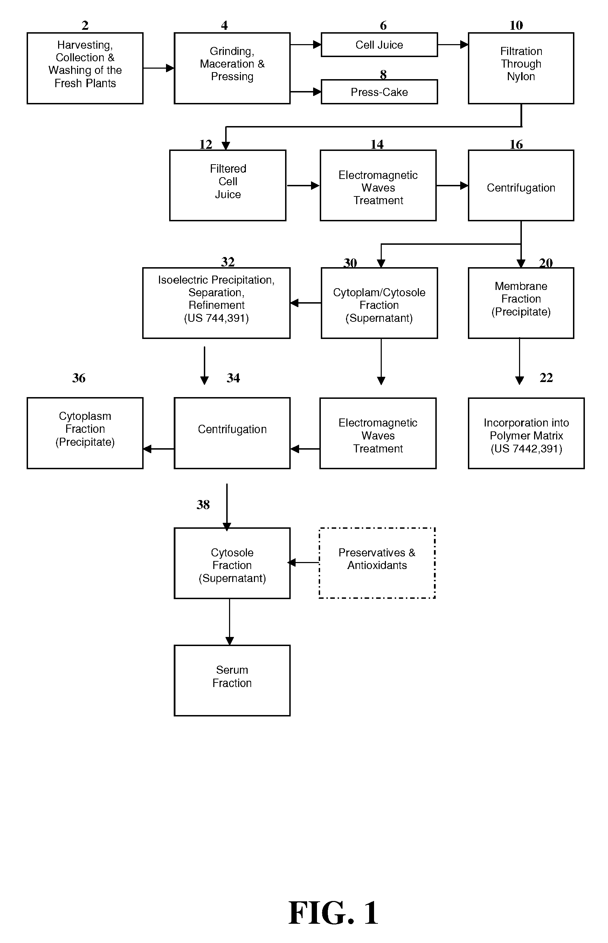

FIG. 1 is a schematic drawing demonstrating one embodiment of the process for preparing the bioactive botanical cosmetic compositions of the present invention.

### Detailed Description of the Invention

The present invention relates to a process for the preparation of botanical fractions from fresh plant biomass according to claim 1 and to compositions made from said fractions according to claim 14 or 15. The process comprises grinding (or maceration) and pressing fresh plant biomass in order to obtain an intracellular plant material, referred to herein as plant cell juice, containing membrane fractions, and treating said cell juice with an electromagnetic waves at a frequency effective to trigger separation of said membrane fraction from said cell juice fraction in order to yield a cell cytoplasm/cytosole fraction substantially-free from membrane fractions. The aforementioned treatment is performed such that the temperature of said cell juice during said treatment does not exceed 40°C.

The membrane fraction can then be utilized in order to provide a stable botanical cosmetic composition exhibiting antiproteolytic, cell growth inhibition activity, and/or both antiproteolytic and cell growth inhibition activities, where the antiproteolytic activity is due to inhibition of at least one proteinase and the cell growth inhibition activity is due to inhibition of cell growth of at least one type of cell.

The cytoplasm/cytosole fraction can be utilized in order to provide a botanical composition suitable for use as a component in a pharmaceutical, cosmetic, nutritional, therapeutic and/or personal care formulation and the like.

### Overall Process for Preparing Botanical Fractions of the Invention

By way of example, the overall process for preparing the bioactive botanical cosmetic compositions of the present invention is described below in reference to FIG. 1. As depicted in FIG. 1, fresh plants are harvested, collected, and washed to yield fresh plant biomass 2. This fresh plant biomass is subjected to grinding, maceration, and pressing 4 to yield intracellular plant material (cell juice) 6 and fiber-enriched material (press-cake) 8. Cell juice 6 is then filtered through nylon mesh 10 to yield filtered plant cell juice 12. Filtered cell juice 12 is exposed to electromagnetic waves treatment 14 at a frequency to trigger its destabilization. The destabilized cell juice is and then subjected to centrifugation 18 in order to yield precipitated membrane fraction 20 and a supernatant which is cytoplasm/cytosole fraction 30. Membrane fraction 20 is a bioactive botanical cosmetic composition which can be added into cosmetic products as described for example, in U.S. Patent Nos. 7,442,391, 8,101,212, 8,277,852 and 8,318,220. Plant cytoplasm/cytosole fraction 30 is used for further processes, as described below.

Cytoplasm/cytosole fraction 30 can optionally be subjected to additional treatments: *i, ii, iii* or *iv.* as summarized below. As a nonlimiting example, treatment (*i*) can include isoelectric precipitation 32 and following centrifugation 34 enabling to separate precipitated cytoplasm fraction 36 from supernatant containing cytosole fraction 38, as described for example, in U.S. Patent Nos. 7,442,391, 8,101,212, and 8,277,852. Alternatively cytosole/cytoplasm fraction can be further separated as result of (ii) additional electromagnetic treatment (at frequency > 7 GHz) with following centrifugation or filtration, or *(iii)* membrane filtration, or (*iv*) ultrafiltration, or combination of thereof (*i, ii, iii, iv*) . Cytoplasm/cytosole fraction components can be utilized "as is" or can be further separated and utilized. They can also be stabilized with preservatives and antioxidants as described for example, in U.S. Patent Nos. 7,442,391; 7,473,435; 7,537,791; 8,043,635; 8,101,212; 8,277,852 and 8,318,220.

### Process for Preparing the Membrane-Derived Cosmetic Compositions

In one embodiment, the process for preparing the Membrane-Derived Cosmetic Compositions is as follows. This method involves providing plant cell juice that has been separated from a fresh plant biomass. "Fresh plant biomass" as it is used throughout this application is intended to mean that a majority of the freshly harvested plant biomass is in the living state and/or it has not undergone a meaningful amount of unwanted degradation. The plant cell juice is then treated under conditions effective to trigger separation it into a membrane fraction and a cell juice supernatant. The resulting membrane fraction has antiproteolytic activity, cell growth inhibition activity, or both antiproteolytic and cell growth inhibition activities. The membrane fraction is then converted under conditions effective to yield a stable bioactive botanical cosmetic composition exhibiting modulation of proteolytic, cell growth inhibition activity, or both proteolytic and cell growth inhibition activities, where the proteolytic activity is due to modulation of at least one proteinase and the cell growth modulation activity is due to modulation of cell growth of at least one type of cell.

The plant cell juice may be separated from all types of plants. Examples of suitable plants that may be used as sources of fresh plant biomass in the present include, without limitation, plants from the following families: *Laminariaceae, Cladophoraceae, Fabeaceae*, *Theaceae, Asteraceae" Lamiaceae, Liliaceae, Poaceae, Moraceae, Apiaceae, Portulacaceae, Rutaceae and Rosaceae.* In particular, examples of specific plants that have been tested and found appropriate as fresh plant biomass sources include Kelp (*Macrocystis pyrifera*), Green Algae (*Chaetomorpha*), Alfalfa (*Medicago saliva*), Red Clover (*Trifolium pratense*)*,* Soy (*Glycine max*), Tea plant (*Camellia sinensis*)*,* Marigold (*Calendula officinalis*)*,* Feverfew (*Tanacetum parthenium*), German Chamomile (*Chamomilla recutita*)*,* Lavender (*Lavandula angustifolia*), Sage (*Salvia officinalis*)*,* Lotus (*Nelumbo nucifera*)*,* Lily (*Lilium bulbiferum*)*,* Oat (*Avena sativa*) and Barley (*Hordeum vulgare*)*,* Ficus species (*Ficus Benghalensis, Ficus carica, Ficus Microcarpa*), Apple (*Pyrus malus*), Dandelion (*Taraxacum officinales*), Lemon (*Citrus limon*), Purslane (*Portulaca oleracea*), Parsley (*Petroselinum crispum*). Various parts of the plants may be used. For example, the stems and leaf tissue may be used for many types of plants. For other plants, the flowers may be used as sources of plant cell juice for use in the present invention. For example, one embodiment of the present invention uses flower tissue of Marigold for the separation of the plant cell juice. In another embodiment, the leaf and stem tissue of Sage is used.

The plant cell juice may be separated using various separation techniques. However, the separation technique resulting in plant cell juice that preserves the bioactive components of the plant.

An exemplary method of preparing the plant biomass for use in extraction of plant cell juice involves harvesting, collecting, and washing of the fresh plants. Suitable steps to follow for preparing the fresh plant biomass include, for example, the following: (1) preservation of the inherent moisture content of the plant cells; (2) optimization of the height of cut used during harvesting of above-ground plant tissue; (3) reservation of plant integrity during harvesting (e.g., during cutting of the above-ground plant tissue); (4) minimization of environmental impact and time factors of biological degradation of the plant biomass; and (5) cleaning of the plant biomass prior to processing (e.g., prior to grinding and maceration). Each of these steps is discussed below.

### Preservation of Inherent Moisture Content:

The cutting should be done to avoid wilting due to moisture loss. Optimal conditions are those where natural moisture content is maintained and preserved.

### Optimal and Preferred Height of Cut:

The plants should be cut at least several centimeters above the ground to limit the amount of soil and other debris in the collected biomass. For example, all useable leaf and stem biomass of any given plant source may be cut at a height of greater than or equal to 5 centimeters above ground. If flower tissue is used as the plant biomass source, the flowers are separated from the whole plant prior to extraction of the plant cell juice.

### Preservation of Plant Integrity During Harvesting:

Harvesting of the plant biomass may be by cutting the above ground stem and leaf tissue of the plant. The cutting is conducted in a manner that avoids or minimizes the chopping, mashing, crushing, or other type of injury of the plant. For large-scale industrial harvesting, where it may not be possible to avoid chopping due to the type of equipment required, care is taken to minimize injury that could lead to microbial growth, moisture loss, intensification of oxidation, polymerization, isomerization, and hydrolysis processes (i.e., unwanted catabolic processes) in collected plants. For example, in one embodiment of the present invention, plants are cut and collected by hand as whole plants. In another embodiment, plant tissue are cut using harvesting equipment. In that case, the minimum chopping height above ground for each plant is greater than or equal to 5 centimeters. Further, particular attention is made to minimize injury during and after cutting. In another embodiment, flowering whole plants are collected by hand and the flowers are then separated for further processing.

### Minimization of Environmental Impact and Time Factors of Degradation:

Delivery time of cut plant material to the processing facility and exposure of biomass to sun, high temperature, and other negative environmental factors, should be minimized to prevent the impact of unwanted degradation processes as described above. For example, in one embodiment of the present invention, the delivery time for *Fabeaceae* plants for further processing does not exceed 30 minutes from the time of cutting. In another embodiment, plants that undergo long distance transport are treated to a post-cutting procedure involving immediately placing the plant biomass into Styrofoam coolers containing bags of frozen gel packs to help maintain freshness and natural moisture content during overnight delivery to the processing facility. These procedures were conducted for plant biomass from *Lamiaceae* and *Moraceae* families. Other post-cutting procedures that achieve the results described above may be used as well. As a nonlimiting example, for many plant species it is beneficial to not only minimize delivery time for processing, but to also keep the cut plant material cool, by refrigeration if necessary, to prevent and/or minimize unwanted degradation prior to and/or during processing.

### Cleaning Step Prior to Grinding and Maceration:

A washing step to remove the soil particles and other debris from plants prior to further processing is performed once the plant tissue is harvested. The washing is achieved using a low-pressure rinse for a short duration under conditions to prevent the initiation of the release of the cell juice from biomass, to cause injury, or to remove valuable components. For example, in one embodiment of the present invention, the washing of the plant biomass was accomplished in less than or equal to 5 minutes with a water pressure of less than or equal to 1 kg/cm². Residual water wash did not contain any green or yellow pigments, which indicates the absence of subsequent injury. The excess water is removed from washed biomass in order to keep the dry matter content close to natural level.

After the plant tissue biomass is harvested, as described above, further processing of the plant tissue biomass is performed to yield plant cell juice. In one embodiment, the harvested plant tissue biomass is subjected to grinding, maceration, and pressing to separate the intracellular content, i.e., the cell juice, and to separate it from the fiber-enriched press-cake containing predominantly cell walls.

An example of a suitable processing protocol involves the steps described below. A hammer mill may be used to grind plants to yield plant tissue particles of a small size in a short time and without significant increase of biomass temperature. In one embodiment, a modified hammer mill is used to produce the maximum size of macerated plant particles less than or equal to 0.5 centimeters during less than or equal to 10 seconds of treatment, where the increase of biomass temperature is less than or equal to 5° C.

Exposure of ground and macerated plant biomass is minimized to prevent the impact of unwanted catabolic processes, as described above. The separation of plant cell juice from fiber-enriched material (or press-cake) is commenced as soon as possible after grinding and maceration of the plant biomass. The plant biomass is processed in a short time and without significant increase in temperature. In one embodiment, immediately after grinding and maceration, the plant biomass is pressed using a horizontal, continuous screw press (Compact Press "CP-6", Vincent Corporation, FL). The pressure on the cone is maintained at level 24 kg/cm², screw speed is at 12 rpm, and biomass temperature increase is less than or equal to 5°C. The initial cell juice usually contains small fiber particles, which can absorb valuable cell juice components and also block the hoses and pumps. The above particles should be removed by filtration or low-speed centrifugation. For example, the initial cell juices produced after the pressing step are filtered through four layers of nylon fabric prior to using the plant cell juice in the methods of the present invention.

Once plant cell juice is separated, the plant cell juice is relatively stable colloidal dispersion in which organelles represent the dispersed phase and cytoplasm represents the continuous phase. Cell juice is then treated to a processes involving (1) triggering destabilization of above colloidal dispersion performing a "initiation of membrane fraction aggregation step" to yield a destabilized cell juice and (2) performing a "membrane fraction separation step" on destabilized cell juice mixture to yield a membrane fraction (containing nucleous, or chloroplasts, or chromoplasts, or mitochondria, or combination of thereof) and a cell juice supernatant. In one embodiment, initiation of membrane fraction destabilization is accomplished by subjecting said cell juice to electromagnetic waves at a frequency of greater than 3 GHz. After destabilization is achieved, a membrane fraction separation step is performed. This step includes, for example, separating of destabilized cell juice into the membrane fraction and the cell juice supernatant using separating techniques including filtration, or centrifugation, or combination of thereof.

A variety of instruments can be employed in the process of the invention in order to generate the electromagnetic waves necessary to destabilize the cell juice: magnetrons, power grid tubes, klystrons, klystrodes, crossed-field amplifier, travelling wave tubes, and gyrotrons. One such instrument includes, but is not limited to high power magnetron. Conventional and industrial magnetrons operate at a frequency of 915 MHz and 2.45 GHz. However at those frequencies undesirable heat is generated that can denature the cell juice composition. In the process of the present invention, the electromagnetic waves operate at frequencies that are substantial higher than the frequencies of conventional or industrial magnetrons, which allows for destabilization of the cell juice without undesirable denaturing due to heat generation. The frequency of said electromagnetic waves in the destabilization step of the present invention is above the frequency of conventional microwave magnetrons, i.e., above 3 GHz, in another embodiment greater than 3 GHz and less than about 7 GHz; and in another embodiment from about 3 to about 6 GHz. During the destabilizing step of the invention the temperature of the cell juice is maintained below 40°C, in another embodiment below about 35°C, in another embodiment below about 30°C, in another embodiment below about 25°C, in another embodiment below about 20°C.

The freshly obtained membrane fraction commonly referred to in the art, as "protein-vitamin concentrate," is a paste having intensive color and specific odor that is plant raw material source specific. The membrane fraction is represented predominantly by chloroplasts present in the green parts of plant or mostly by chromoplasts present in flowers. The composition of the membrane fraction includes predominantly phospholipids, membrane proteins, chlorophyll, nucleus, mitochondria and carotenoids.

### Process for Preparing Cytoplasm/Cytosole Fraction Derived Cosmetic Compositions Substantially-Free From Membrane Fractions

The present invention also relates to a method for preparing the cytoplasm/cytosole fraction derived cosmetic compositions substantially-free from membrane fractions exhibiting antioxidant activity, cell growth stimulation activity, or both antioxidant and cell growth stimulation activities. The method involves providing a cell juice that has been separated from a fresh plant biomass, as already described above with respect to the Membrane-Derived Cosmetic Composition. The plant cell juice is then treated under conditions effective to separate the plant cell juice into a membrane fraction and a cytoplasm/cytosole fraction.

The cytoplasm/cytosole fraction can then be optionally further processed under conditions effective to separate the cytoplasm/cytosole fraction into its component parts, namely the cytoplasm fraction and a cytosole fraction. The cytoplasm fraction includes predominantly white soluble proteins; in C3 plants, these proteins largely consist of the enzyme ribulose-1,5biphosphate carboxylase oxygenase. The cytosole fraction contains low molecular weight soluble components. Cytosole fraction is refined under conditions effective to yield a cell serum fraction having antioxidant activity, cell growth stimulation activity, or both antioxidant and cell growth stimulation activities. The cell serum fraction is stabilized under conditions effective to yield a stable bioactive botanical cosmetic composition exhibiting antioxidant activity, cell growth stimulation activity, or both antioxidant and cell growth stimulation activities as described for example, in US patents 7,442,391; 7,473,435; 7,537,791; 8,043,635; 8,101,212; 8,277,852 and 8,318,220.

The plant cell juice may be obtained from all types of plants. Examples of suitable plants that may be used as sources of fresh plant biomass in the present include, without limitation, plants from the following families: *Laminariaceae, Cladophoraceae, Fabeaceae*, *Theaceae, Asteraceae" Lamiaceae, Liliaceae, Poaceae, Moraceae, Apiaceae, Portulacaceae, Rutaceae and Rosaceae.* In particular, examples of specific plants that have been tested and found appropriate as fresh plant biomass sources include Kelp (*Macrocystis pyrifera*), Green Algae (*Chaetomorpha*), Alfalfa (*Medicago saliva*), Red Clover (*Trifolium pratense*), Soy (*Glycine max*), Tea plant (*Camellia sinensis*), Marigold (*Calendula officinalis*), Feverfew (*Tanacetum parthenium*), German Chamomile (*Chamomilla recutita*), Lavender (*Lavandula angustifolia*), Sage (*Salvia officinalis*), Lotus (*Nelumbo nucifera*), Lily (*Lilium bulbiferum*), Oat (*Avena sativa*) and Barley (*Hordeum vulgare*), Ficus species (*Ficus benghalensis, Ficus carica, Ficus microcarpa*), Apple (*Pyrus malus*), Dandelion (*Taraxacum officinales*)*,* Lemon (*Citrus limon*)*,* Purslane (*Portulaca oleracea*), Parsley (*Petroselinum crispum*). Various parts of the plants may be used. For example, the stems and leaf tissue may be used for many types of plants. For other plants, the flowers may be used as sources of plant cell juice for use in the present invention. For example, one embodiment of the present invention uses flower tissue of Marigold for the separation of the plant cell juice. In another embodiment, the leaf and stem tissue of Sage is used. As described above, once the plant cell juice is separated into membrane fraction and a cell juice supernatant, i.e. cytoplasm/cytosole fraction 30 which is subjected to additional treatments: *i, ii, iii* or *iv* (FIG. 1) enabling to separate cytoplasm fraction from cytosole fraction.

The quantitative criteria to evaluate the complete separation of cytoplasm fraction is the absence of detectable levels of high molecular weight proteins and/or the absence of ribulose-1,5-biphosphate carboxilase oxygenase in cytosole fraction.

The cytosole fraction is clear liquid which has a slight yellow color and slight characteristic odor. In several hours, the unstable cytosole fraction is irreversibly transformed into dark brown color suspension containing heavy precipitate and strong non-characteristic odor. As a result, cytosole fraction cannot be used as a cosmetic ingredient. The described procedure that follows allows for the refinement of cytosole fraction to yield stable and active serum fraction which is stable cosmetic ingredients. This is accomplished by removing from cytosole fraction the major components responsible for the irreversible transformations that lead to generation of unwanted precipitate and deterioration of color and odor. This procedure includes: pH adjustment, heat treatment, cooling, vacuum filtration, and stabilization as described in U.S. Patent Nos. 7,442,391, 8,101,212, 8,277,852 and 8,318,220. After the cell serum fraction is produced, it is then subjected to the stabilizing step to yield the Serum-Derived Cosmetic Composition. In one embodiment, the stabilizing step involves incubating the cell serum fraction in a mixture of at least one preservative and at least one antioxidant to yield a stabilized cell serum fraction. Suitable preservatives for use in the present invention include, for example, potassium sorbate, sodium benzoate, sodium methyl paraben, and citric acid. An example of a suitable antioxidant for use in the present invention is sodium metabisulfite.

### EXAMPLES

Although preferred embodiments have been depicted and described in detail herein, it will be apparent to those skilled in the relevant art that various modifications, additions, substitutions, and the like can be made within the scope of the invention as defined in the claims which follow.

### Example 1 (Frequencies),

Separation of Cell Juices from fresh Parsley (*Petroselinum Crispum*), Dandellion (*Traxacam Officinalis*)*,* Feverfew (*Chrysanthemum Parthenium*), German Chamomile (*Chamomilla Recutita*)*,* Marigold (*Calendula Offiinalis*)*,* Alfalfa (*Medicago Sativa*), Red Clover (*Trifolium Pratense*), Soy (*Glycine Max*), Lavender (*Lavandula Angustifolia*)*,* Sage (*Salvia Officinale*)*,* Kelp (*Macrosystis Pyrifera*), Lily (*Lilium Bulbiferum*), Lotus (*Nelumbo Nucifera*), Ficus Benghalensis (*Ficus Benghalensis*)*,* Ficus Carica (*Ficus Carica*)*,* FIcus Microcarpa (*Ficus Microcarpa),* Barley (*Hordeum Vulgare*)*,* Oat (*Avena Saliva*)*,* Purslane (*Portulaca Oleraceae*), Apple (*Pyrus Malus*), Lemon (*Citrus Limon*)*,* Tea Plant (*Camellia Sinensis*). Cell juices were prepared as described in U.S. Patent No 7,442,391. These cell juices represented colloidal dispersion which remained their stability after low speed centrifugation (3,000 g x 20 minutes). These cell juices were exposed to magnetron pulses treatment using broadband dielectric spectrometer control. The treatment continued until cell juice was destabilized, i.e. became separable by low-speed centrifugation (3,000 g x 20 min) to precipitate (membrane fraction) and corresponding chlorophyll free transparent supernatant (cytoplasm/cytosole fraction). During above treatment and following separation, temperature of cell juice was ≤ 37°C. Experimental data summary presented in Table 1.

**Table 1.**

| Plant Families | Plant Species | Frequency, GHz |
|---|---|---|
| *Apiacaea* | Parsley | ≥ 5.3 |
| *Asteraceae* | Dandellion | ≥ 5.0 |
| | Feverfew | ≥ 6.0 |
| | German Chamomile | ≥ 3.8 |
| | Marigold | TBD* |
| *Fabaceae* | Alfalfa | ≥ 4.0 |
| | Red Clover | ≥ 4.0 |
| | Soy | ≥ 3.5 |
| *Lamiaceae* | Lavender | ≥ 4.0 |
| | Sage | ≥ 5.0 |
| *Laminariaceae* | Kelp | ≥ 10.5 |
| *Liliaceae* | Lily | ≥ 3.5 |
| | Lotus | TBD* |
| *Moraceae* | Ficus Benghalensis | ≥ 4.5 |
| | Ficus Carica | ≥ 5.0 |
| | FIcus Microcarpa | ≥ 4.0 |
| *Poaceae* | Barley | ≥ 4.0 |
| | Oat | ≥ 4.0 |
| *Portulacaceae* | Purslane | ≥ 5.0 |
| *Rosaceae* | Apple | ≥ 7.0 |
| *Rutaceae* | Lemon | ≥ 8.0 |
| *Theaceae* | Tea Plant | ≥ 4.0 |

| | | |
|---|---|---|
| *) - to be determined. There are no reliable experimental data yet. | | |

The cell juice supernatant (cytoplasm/cytosole fraction) was used for further processing.

### Example 2 (Dielectric Constant Decrease).

Separation of Cell Juices from fresh Parsley (*Petroselinum Crispum*), Dandellion (*Traxacam Officinalis*)*,* Feverfew (*Chrysanthemum Parthenium*), German Chamomile (*Chamomilla Recutita*)*,* Marigold (*Calendula Offiinalis*), Alfalfa (*Medicago Sativa*), Red Clover (*Trifolium Pratense*), Soy (*Glycine Max*), Lavender (*Lavandula Angustifolia*), Sage (*Salvia Officinale*), Kelp (*Macrosystis Pyrifera*)*,* Lily (*Lilium Bulbiferum*)*,* Lotus (*Nelumbo Nucifera*)*,* Ficus Benghalensis (*Ficus Benghalensis*), Ficus Carica (*Ficus Carica*), FIcus Microcarpa (*Ficus Microcarpa*)*,* Barley (*Hordeum Vulgare*), Oat (*Avena Sativa*), Purslane (*Portulaca Oleraceae*), Apple (*Pyrus Malus*), Lemon (*Citrus Limon*)*,* Tea Plant (*Camellia Sinensis*). Cell juices were prepared as described in U.S. Patent No. 7,442,391 . These cell juices represented colloidal dispersion which remained their stability after low speed centrifugation (3,000 g x 20 minutes). These cell juices were exposed to short term (from 10 seconds to 1 minute) magnetron pulses treatment using broadband dielectric spectrometer control. The treatment continued until certain decrease was achieved in the value of dielectric constant (Δε₀) extrapolated from Cole-Cole plot at low frequency and minimum dielectric loses of applied field. The values of magnetron frequencies and Δε₀ required for separation of cell juice to membrane fraction and corresponding cytoplasm/cytosole fraction are presented in Table 2. At these conditions low speed centrifugation (3,000 g x 20 minutes) of cell juices was found to be sufficient for complete separation of chlorophyll containing precipitate (membrane fraction) from chlorophyll free transparent supernatant (cytoplasm/cytosole fraction). During above treatment and following separation, temperature of cell juice was ≤ 37°C. Experimental data summary presented in Table 2.

**Table 2.**

| Plant Families | Plant Species | Frequency, GHz | Δ□□, F/m |
|---|---|---|---|
| Apiacaea | Parsley | ≥ 5.3 | 4 |
| Asteraceae | Dandellion | ≥ 5.0 | 4 |
| | Feverfew | ≥ 6.0 | 6 |
| | German Chamomile | ≥ 3.8 | 5 |
| | Marigold | TBD* | TBD* |
| Fabaceae | Alfalfa | ≥ 4.0 | 9 |
| | Red Clover | ≥ 4.0 | 4 |
| | Soy | ≥ 3.5 | 4 |
| Lamiaceae | Lavender | ≥ 4.0 | 2 |
| | Sage | ≥ 5.0 | 3 |
| Laminariaceae | Kelp | ≥ 10.5 | 10 |
| Liliaceae | Lily | ≥ 3.5 | 6 |
| | Lotus | TBD* | TBD* |
| Moraceae | Ficus Benghalensis | ≥ 4.5 | 5 |
| | Ficus Carica | ≥ 5.0 | 4 |
| | FIcus Microcarpa | ≥ 4.0 | 5 |
| Poaceae | Barley | ≥ 4.0 | 4 |
| | Oat | ≥ 4.0 | 4 |
| Portulacaceae | Purslane | ≥ 5.0 | 4 |
| Rosaceae | Apple | ≥ 7.0 | 9 |
| Rutaceae | Lemon | ≥ 8.0 | 12 |
| Theaceae | Tea Plant | ≥ 4.0 | 5 |

| | | | |
|---|---|---|---|
| *) - to be determined. There are no reliable experimental data yet. | | | |

The cell juice supernatant (cytoplasm/cytosole fraction) was used for further processing.

### Example 3 (Surface Potential Decrease).

Separation of Cell Juices from fresh Parsley (*Petroselinum Crispum*), Dandellion (*Traxacam Officinalis*)*,* Feverfew (*Chrysanthemum Parthenium*)*,* German Chamomile *(Chamomilla Recutita*), Marigold (*Calendula Offiinalis*)*,* Alfalfa (*Medicago Sativa*), Red Clover (*Trifolium Pratense*), Soy *(Glycine Max*), Lavender (*Lavandula Angustifolia*)*,* Sage (*Salvia Officinale*)*,* Kelp (*Macrosystis Pyrifera*)*,* Lily (*Lilium Bulbiferum*)*,* Lotus (*Nelumbo Nucifera*), Ficus Benghalensis (*Ficus Benghalensis*)*,* Ficus Carica (*Ficus Carica*)*,* FIcus Microcarpa (*Ficus Microcarpa),* Barley (*Hordeum Vulgare*), Oat (*Avena Sativa*), Purslane ( *Portulaca Oleraceae*), Apple (*Pyrus Malus*), Lemon (*Citrus Limon*)*,* Tea Plant (*Camellia Sinensis*)*.* Cell juices were prepared as described in US patent 7,442,391. These cell juices represented colloidal dispersion which remained their stability after low speed centrifugation (3,000 g x 20 minutes). These cell juices were exposed to short term (from 10 seconds to 1 minute) magnetron pulses treatment using broadband dielectric spectrometer control. The treatment continued until certain decrease was achieved in the value of surface potential of cell juice measured with Kelvin Probe vibro-capacitor. The values of magnetron frequencies and decrease of Surface Potential value (ΔSP) required for separation of cell juice to membrane fraction and corresponding cytoplasm/cytosole fraction are presented in Table 3. At these conditions low speed centrifugation (3,000 g x 20 minutes) of cell juices was found to be sufficient for complete separation of chlorophyll containing precipitate (membrane fraction) from chlorophyll free transparent supernatant (cytoplasm/cytosole fraction). During above treatment and following separation, temperature of cell juice was ≤ 37°C. Experimental data summary presented in Table 3.

**Table 3**

| Plant Families | Plant Species | Frequency, GHz | ΔSP, mV |
|---|---|---|---|
| Apiacaea | Parsley | **≥** 5.3 | 111 |
| Asteraceae | Dandellion | **≥** 5.0 | 248 |
| | Feverfew | **≥** 6.0 | 10 |
| | German Chamomile | **≥** 3.8 | 30 |
| | Marigold | TBD* | 201 |
| Fabaceae | Alfalfa | **≥** 4.0 | 35 |
| | Red Clover | **≥** 4.0 | 8 |
| | Soy | **≥** 3.5 | 13 |
| Lamiaceae | Lavender | **≥** 4.0 | 16 |
| | Sage | **≥** 5.0 | 10 |
| Laminariaceae | Kelp | **≥** 10.5 | 59 |
| Liliaceae | Lily | **≥** 3.5 | 33 |
| | Lotus | TBD* | 34 |
| Moraceae | Ficus Benghalensis | **≥** 4.5 | 16 |
| | Ficus Carica | **≥** 5.0 | 56 |
| | FIcus Microcarpa | **≥** 4.0 | 51 |
| Poaceae | Barley | **≥** 4.0 | 52 |
| | Oat | **≥** 4.0 | 45 |
| Portulacaceae | Purslane | **≥** 5.0 | 19 |
| Rosaceae | Apple | **≥** 7.0 | 74 |
| Rutaceae | Lemon | **≥** 8.0 | 168 |
| Theaceae | Tea Plant | **≥** 4.0 | 25 |

The cell juice supernatant (cytoplasm/cytosole fraction) was used for further processing.

## Claims

1. A method for the preparation of a botanical fraction from plant cell juice derived from fresh plant biomass, wherein the process comprises subjecting said plant cell juice to an electromagnetic field at a frequency of greater than 3 GHz for a time effective to destabilize the plant cell juice yielding a coagulated cell juice mixture comprising a coagulated membrane fraction, and separating said coagulated membrane fraction from said coagulated cell juice mixture in order to yield a bioactive fraction comprising a cytoplasm/cytosole fraction that is substantially-free from said membrane fraction, wherein the temperature of said cell juice is maintained at or below 40°C.

2. The method according to claim 1, wherein said plant cell juice is destabilized by subjecting said plant cell juice to an electromagnetic field at a frequency of from greater than 3 GHz to 7.0 GHz.

3. The method according to claim 2, wherein said electromagnetic field is generated by a high power magnetron.

4. The method according to claim 1, wherein said separation of said coagulated membrane fraction is carried out by filtration or centrifugation.

5. The method according to claim 2, wherein the frequency of said electromagnetic field is from greater than 3.0 GHz to 6.0 GHz.

6. The method of claim 5, wherein the temperature of said cell juice is maintained at or below 30°C.

7. The method of claim 6, wherein the temperature of said cell juice is maintained at or below 25°C.

8. The method according to claim 1, wherein said cytoplasm/cytosole fraction is stabilized by incubating said cytoplasm/cytosole fraction in a mixture of at least one preservative and at least one antioxidant to yield the stable bioactive botanical cosmetic composition.

9. The method according to claim 8, wherein said preservative is selected from the group consisting of potassium sorbate, sodium benzoate, sodium methyl paraben, and citric acid.

10. The method according to claim 8, wherein said antioxidant is sodium metabisulfite.

11. The method according to claim 1, wherein said fresh plant biomass is derived from a plant family selected from *Laminariaceae, Cladophoraceae, Fabeaceae, Teaceae, Asteraceae" Lamiaceae, Liliaceae, Poaceae* and *Moraceae.*

12. The method of claim 1 wherein said fresh plant biomass is selected from the group consisting of *Macrocystis porifera, Chaetomorpha, Medicago sativa, Trifolium pratense, Glycine max, Camellia sinensis, Calendula officinalis, Tanacetum parthenium, Chamomilla recutita , Lavandula angustifolia, Salvia officinalis, Nelumbo nucifera, Lilium bulbiferum, Avena sativa* and *Hordeum vulgare*

13. The method of claim 1 wherein said bioactive fraction comprises coagulated membrane fraction.

14. A stable bioactive botanical cosmetic composition comprising an effective amount of the cytoplasm/cytosole fraction that is substantially-free from membrane fraction of claim 1.

15. A stable bioactive botanical cosmetic composition comprising an effective amount of the membrane fraction of claim 1.

16. The method of claim 1 wherein said method is substantially free of added extraneous solvent, substantially free of added extraneous water, or substantially free of both added extraneous solvent and added extraneous water

17. The method of claim 16 wherein said method is free of added extraneous solvent and added water.

18. The method according to claim 5, wherein the frequency of said electromagnetic field is from greater than 3.5 GHz to 6.0 GHz.

## Patentansprüche

1. Ein Verfahren zur Herstellung einer botanischen Fraktion aus Pflanzenzellsaft abgeleitet aus frischer Pflanzenbiomasse,
wobei das Verfahren eine Behandlung des Pflanzenzellsafts mit einem elektromagnetischen Feld bei einer Frequenz von größer als 3 GHz für einen Zeitraum, der den Pflanzenzellsaft wirksam destabilisiert, um eine koagulierte Zellsaftmischung umfassend eine koagulierte Membranfraktion zu erhalten, und eine Trennung der koagulierten Membranfraktion von der koagulierten Zellsaftmischung, um eine bioaktive Fraktion enthaltend eine Zytoplasma/Zytosol-Fraktion zu erhalten, die im Wesentlichen frei von der Membranfraktion ist,
wobei die Temperatur des Zellsaftes bei oder unterhalb 40°C gehalten wird, umfasst.

2. Das Verfahren gemäß Anspruch 1, wobei der Pflanzenzellsaft destabilisiert wird, indem der Pflanzenzellsaft mit einem elektromagnetischen Feld bei einer Frequenz von größer als 3 GHz bis 7,0 GHz behandelt wird.

3. Das Verfahren gemäß Anspruch 2, wobei das elektromagnetische Feld durch ein Hochleistungs-Magnetron erzeugt wird.

4. Das Verfahren gemäß Anspruch 1, wobei die Trennung der koagulierten Membranfraktion durch Filtration oder Zentrifugieren durchgeführt wird.

5. Das Verfahren gemäß Anspruch 2, wobei die Frequenz des elektromagnetischen Feldes größer als 3,0 GHz bis 6,0 GHz ist.

6. Das Verfahren gemäß Anspruch 5, wobei die Temperatur des Zellsaftes bei oder unterhalb 30°C gehalten wird.

7. Das Verfahren gemäß Anspruch 6, wobei die Temperatur des Zellsaftes bei oder unterhalb 25°C gehalten wird.

8. Das Verfahren gemäß Anspruch 1, wobei die Zytoplasma/Zytosol-Fraktion durch Inkubation der Zytoplasma/Zytosol-Fraktion in einer Mischung von wenigstens einem Konservierungsmittel und wenigstens einem Antioxidationsmittel stabilisiert wird, um die stabile bioaktive botanische kosmetische Zusammensetzung zu ergeben.

9. Das Verfahren gemäß Anspruch 8, wobei das Konservierungsmittel ausgewählt wird aus der Gruppe bestehend aus Kaliumsorbat, Natriumbenzoat, Natriummethylparaben und Citronensäure.

10. Das Verfahren gemäß Anspruch 8, wobei das Antioxidationsmittel Natriummetabisulfit ist.

11. Das Verfahren gemäß Anspruch 1, wobei die frische Pflanzenbiomasse abgeleitet wird von einer Pflanzenfamilie ausgewählt aus *Laminariaceae, Cladophoraceae, Fabeaceae, Teaceae, Asteraceae, Lamiaceae, Liliaceae, Poaceae* und *Moraceae.*

12. Das Verfahren gemäß Anspruch 1, wobei die frische Pflanzenbiomasse ausgewählt wird aus der Gruppe bestehend aus *Macrocystis porifera, Chaetomorpha, Medicago sativa, Trifolium pratense, Glycine max, Camellia sinensis, Calendula officinalis, Tanacetum parthenium, Chamomilla recutita, Lavandula angustifolia, Salvia officinalis, Nelumbo nucifera, Lilium bulbiferum, Avena sativa* und *Hordeum vulgare.*

13. Das Verfahren gemäß Anspruch 1, wobei die bioaktive Fraktion koagulierte Membranfraktion umfasst.

14. Eine stabile bioaktive botanische kosmetische Zusammensetzung umfassend eine wirksame Menge der Zytoplasma/Zytosol-Fraktion, die im Wesentlichen frei von Membranfraktion aus Anspruch 1 ist.

15. Eine stabile bioaktive botanische kosmetische Zusammensetzung umfassend eine wirksame Menge der Membranfraktion aus Anspruch 1.

16. Das Verfahren gemäß Anspruch 1, wobei das Verfahren im Wesentlichen frei von hinzugefügtem fremden Lösungsmittel, im Wesentlichen frei von hinzugefügtem fremden Wasser oder im Wesentlichen frei von sowohl hinzugefügtem fremden Lösungsmittel als auch hinzugefügtem fremden Wasser ist.

17. Das Verfahren gemäß Anspruch 16, wobei das Verfahren frei von hinzugefügtem fremden Lösungsmittel und hinzugefügtem Wasser ist.

18. Das Verfahren gemäß Anspruch 5, wobei die Frequenz des elektromagnetischen Feldes größer als 3,5 GHz bis 6,0 GHz ist.

## Revendications

1. Procédé pour la préparation d'une fraction végétale à partir de jus cellulaire végétal issu de biomasse végétale fraîche, le procédé comprenant l'exposition dudit jus cellulaire végétal à un champ électromagnétique à une fréquence supérieure à 3 GHz pendant une durée efficace pour déstabiliser le jus cellulaire végétal en donnant un mélange de jus cellulaire végétal coagulé comprenant une fraction de membranes coagulée, et la séparation de ladite fraction de membranes coagulée d'avec ledit mélange de jus cellulaire coagulé afin d'obtenir une fraction bioactive comprenant une fraction de cytoplasme/cytosol qui est pratiquement exempte de ladite fraction de membranes, la température dudit jus cellulaire étant maintenue à ou au-dessous de 40 °C.

2. Procédé selon la revendication 1, dans lequel ledit jus cellulaire végétal est déstabilisé par exposition dudit jus cellulaire végétal à un champ électromagnétique à une fréquence de plus de 3 GHz à 7,0 GHz.

3. Procédé selon la revendication 2, dans lequel ledit champ électromagnétique est engendré par un magnétron à haute puissance.

4. Procédé selon la revendication 1, dans lequel ladite séparation de ladite fraction de membranes coagulée est effectuée par filtration ou centrifugation.

5. Procédé selon la revendication 2, dans lequel la fréquence dudit champ électromagnétique est de plus de 3,0 GHz à 6,0 GHz.

6. Procédé selon la revendication 5, dans lequel la température dudit jus cellulaire est maintenue à ou au-dessous de 30 °C.

7. Procédé selon la revendication 6, dans lequel la température dudit jus cellulaire est maintenue à ou au-dessous de 25 °C.

8. Procédé selon la revendication 1, dans lequel ladite fraction de cytoplasme/cytosol est stabilisée par incubation de ladite fraction de cytoplasme/cytosol dans un mélange d'au moins un conservateur et d'au moins un antioxydant pour donner la composition cosmétique végétale bioactive stable.

9. Procédé selon la revendication 8, dans lequel ledit conservateur est choisi dans le groupe constitué par le sorbate de potassium, le benzoate de sodium, le méthylparabène sodique et l'acide citrique.

10. Procédé selon la revendication 8, dans lequel ledit antioxydant est le métabisulfite de sodium.

11. Procédé selon la revendication 1, dans lequel ladite biomasse végétale fraîche est issue d'une famille de plantes choisie parmi les *Laminariaceae, Cladophoraceae, Fabeaceae, Teaceae, Asteraceae, Lamiaceae, Liliaceae, Poaceae* et *Moraceae.*

12. Procédé selon la revendication 1, dans lequel ladite biomasse végétale fraîche est choisie dans le groupe constitué par *Macrocystis porifera, Chaetomorpha, Medicago sativa, Trifolium pratense, Glycine max, Camellia sinensis, Calendula officinalis, Tanacetum parthenium, Chamomilla recutita, Lavandula angustifolia, Salvia officinalis, Nelumbo nucifera, Lilium bulbiferum, Avena sativa* et *Hordeum vulgare.*

13. Procédé selon la revendication 1, dans lequel ladite fraction bioactive comprend une fraction de membranes coagulée.

14. Composition cosmétique végétale bioactive stable comprenant une quantité efficace de la fraction de cytoplasme/cytosol qui est pratiquement exempte de fraction de membranes de la revendication 1.

15. Composition cosmétique végétale bioactive stable comprenant une quantité efficace de la fraction de membranes de la revendication 1.

16. Procédé selon la revendication 1, ledit procédé étant pratiquement exempt de solvant étranger ajouté, pratiquement exempt d'eau étrangère ajoutée ou pratiquement exempt à la fois de solvant étranger ajouté et d'eau étrangère ajoutée.

17. Procédé selon la revendication 16, le procédé étant exempt de solvant étranger ajouté et d'eau ajoutée.

18. Procédé selon la revendication 5, dans lequel la fréquence dudit champ électromagnétique est de plus de 3,5 GHz à 6,0 GHz.
